# FASCICULE DE BREVET EUROPEEN

(11) **EP 0 618 183 B1**
(45) Date de publication et mention de la délivrance du brevet: **18.06.1997**
(21) Numéro de dépôt: 94400677.4
(22) Date de dépôt: 30.03.1994
(51) Int. Cl.: C07C 51/12, C07C 51/10, C07C 67/36, C07C 67/37

(54) **Procédé de préparation d'acides carboxyliques ou des esters correspondants en présence d'un catalyseur à base de rhodium et d'iridium**
Verfahren zur Herstellung von Carbonsäuren oder ihrer Ester in Gegenwart des auf Rhodium und Iridium basierten Katalysators
Process for the preparation of carboxylic acids or corresponding esters in presence of a catalyst based on rhodium and iridium

(30) Priorité: 31.03.1993 FR 9303733
(43) Date de publication de la demande: 05.10.1994
(73) Titulaire: PARDIES ACETIQUES, 92082 Paris La Défense 2 (FR)
(72) Inventeur: Nobel, Dominique, F-69270 Fontaines/Saint/Martin (FR); Perron, Robert, F-69390 Charly (FR); Denis, Philippe, F-69150 Decines (FR)
(74) Mandataire: Portal, Gérard

(56) Documents cités:
- GB-A- 2 142 012
- PATENT ABSTRACTS OF JAPAN, vol. 9, no. 96 (C-278)(1819), 25 avril 1985 & JP-A-59 227 832
- PATENT ABSTRACTS OF JAPAN, vol. 9, no. 96 (C-278)(1819), 25 avril 1985 & JP-A-59 227 831
- PATENT ABSTRACTS OF JAPAN, vol. 9, no. 222 (C-302)(1945), 9 septembre 1985 & JP-A-60 084 234

## Description

La présente invention concerne un procédé de préparation d'acides carboxyliques comprenant (n+1) atomes de carbone, par réaction de monoxyde de carbone avec au moins un composé carbonylable présentant (n) atomes de carbone, en présence d'un catalyseur homogène à base de rhodium et d'iridium.

L'obtention d'acides carboxyliques, et plus particulièrement d'acide acétique, par réaction d'un alcool, comme le méthanol, avec du monoxyde carbone, est un procédé bien connu, exploité industriellement, et ayant fait l'objet de nombreux articles et brevets, comme par exemple US 3 769 329, US 3 813 428. Les procédés décrits dans ces références mettent en oeuvre un système catalytique à base de rhodium et d'un composé halogéné,

De tels procédés sont habituellement exploités dans des installations constituées d'une zone de réaction comprenant un réacteur sous pression, d'une zone de séparation de l'acide formé, du reste de mélange réactionnel la séparation étant réalisée par vaporisation partielle dudit mélange. La partie vaporisée, comprenant principalement l'acide produit, est ensuite envoyée dans une zone de purification, constituée de plusieurs colonnes de distillation ; l'autre partie, comprenant notamment le système catalytique, reste sous forme liquide et est recyclée au réacteur.

Malgré leurs performances reconnues, ces modes de préparation présentent cependant des contraintes importantes dans la conduite de la réaction. Celles-ci sont dues principalement à la nature du catalyseur mis en oeuvre, et plus particulièrement à la nature de l'élément métallique du système catalytique. Comme indiqué précédemment, le métal utilisé industriellement dans la réaction de carbonylation du méthanol est le rhodium. Celui-ci, dans les réactions en phase liquide, se présente classiquement sous la forme d'un complexe soluble de rhodium dont les ligands sont du monoxyde de carbone et de l'iode.
Or il est maintenant reconnu que de tels catalyseur sont extrêmement sensibles à des variations de composition du milieu réactionnel, comme notamment la teneur en eau, ou encore la pression partielle en monoxyde de carbone. Une baisse de la teneur de l'un de ces constituants ou des deux, dans le milieu réactionnel, peut être la cause d'une perte de rhodium sous la forme d'un précipité insoluble et inactif, entraînant par voie de conséquence, une baisse de la productivité de la réaction.

La zone critique du procédé, en ce qui concerne la déficience en monoxyde de carbone, se situe dans la zone de séparation où le mélange réactionnel est partiellement vaporisé sous l'effet d'une détente.

La pression partielle en CO est alors considérablement diminuée, entraînant des pertes irréversibles en métal précieux.

Plusieurs solutions ont été préconisées pour compenser l'effet de la déficience en monoxyde de carbone. Une solution évidente est l'addition supplémentaire de rhodium, mais un tel moyen n'est pas économiquement viable.

D'autres moyens plus satisfaisants ont été proposés, comme par exemple une augmentation de la teneur en eau, ou l'ajout de stabilisants dans le milieu réactionnel. La demande de brevet EP 55618 notamment, décrit l'utilisation de tels composés. Ces derniers peuvent être choisis parmi des espèces organiques comme le N,N,N',N'-tétraméthyl-o-phénylène diamine, les phosphines disubstituées comme le bis(diphénylphosphino) méthane, le bis (di-p-tolylphosphino) méthane, les polyacides comme l'acide citrique, l'acide succinique ou encore parmi des composés minéraux à base de germanium, d'antimoine, d'étain ou de métaux alcalins, sous forme d'halogénures, d'acétate ou encore d'oxydes.

Quant à la teneur en eau dans le milieu, celle-ci à une influence sur la totalité de l'installation et non pas seulement dans les zones où le catalyseur est présent.

En effet, l'eau a une action bénéfique reconnue sur la stabilité du rhodium mais aussi sur la vitesse de carbonylation, donc sur la productivité de l'installation. C'est pourquoi les premiers procédés développés mettent en oeuvre des compositions réactionnelles présentant de très fortes teneurs en eau, de l'ordre de 14 à 20 % en général, rapportés au poids total du mélange. Cependant, la présence de telles teneurs en eau dans la zone de séparation et de purification de l'acide formé est contraignante en ce sens qu'elle représente une importante quantité d'énergie consommée pour obtenir une déshydratation suffisante de l'acide.

Ainsi, deux intérêts se trouvent en opposition, si l'on ne considère que le facteur relatif à la teneur en eau : le premier a trait à l'obligation de conserver le catalyseur sous une forme soluble dans le milieu pour une bonne productivité de la réaction, donc à augmenter la quantité d'eau, tandis que le second tend à la diminution de celle-ci afin de minimiser le coût de la séparation ultérieure de l'acide formé de l'eau présente.

De nouveaux procédés sont apparus offrant un compromis entre les intérêts contradictoires indiqués.

Ainsi, la demande de brevet EP 161 874, décrit un procédé de carbonylation du méthanol en acide acétique, mettant en oeuvre le système catalytique usuel ; l'innovation apportée consistant à maintenir pendant le cours de la carbonylation, les constituants du mélange réactionnel, précisément définis, dans des pro-portions bien particulières. Le procédé est réalisé en présence d'une teneur en eau inférieure à 14 % et pouvant être aussi faible que 1%, et requiert, par ailleurs, l'utilisation de quantités considérables, jusqu'à 20 %, d'un sel soluble d'iodure stabilisant le rhodium, de préférence l'iodure de lithium.

La présente invention a pour objet un procédé de préparation, en phase liquide, d'acides carboxyliques, mettant en oeuvre un système catalytique à base de rhodium et d'iridium, le procédé selon l'invention permettant d'opérer dans une gamme étendue de teneurs en eau dans le mélange réactionnel, sans que l'on constate par ailleurs une perte en métaux précieux par précipitation, aux faibles teneurs en eau. En outre, le procédé selon l'invention permet d'atteindre des productivités comparables à celles des procédés classiques.

De plus, et ceci représente un avantage totalement inattendu, les résultats mentionnés ci-dessus peuvent être obtenus sans qu'il soit nécessaire de mettre en oeuvre des quantités considérables d'additifs stabilisant le catalyseur. Il a même été constaté que le procédé selon l'invention pouvait être mis en oeuvre de façon efficace, sans avoir à utiliser de tels composés.

Ainsi, le procédé selon l'invention concerne l'obtention d'acides carboxyliques comprenant (n+1) atomes de carbone, ou des esters correspondants, par mise en contact dans le mélange réactionnel, de monoxyde de carbone avec au moins un réactif choisi parmi les composés de formules :

R(OH)ₘ ; (1)

RX ; (2)

ROR' (3)

ROCOR' ; (4)

Formules dans lesquelles R et R', identiques ou différents, représentent chacun un radical hydrocarboné en C₁-C₁₀, R présentant n atomes de carbone ; X représente le chlore, le brome, ou l'iode et m vaut 1 ou 2, la réaction étant effectuée en présence d'un système catalytique comprenant au moins un composé du rhodium, au moins un composé de l'iridium ou au moins un composé renfermant ces deux métaux, et au moins un promoteur halogéné.

Ainsi que cela a été indiqué plus haut, la présente invention est réalisée avec un système catalytique à base de deux métaux: le rhodium et l'iridium.

La conjonction de ces deux éléments apporte deux avantages par rapport à un procédé ne mettant en oeuvre qu'un métal seul.

Il a en effet été trouvé que l'utilisation simultanée de rhodium et d'iridium, comme constituants métalliques du système catalytique, permettait d'augmenter d'une façon totalement inattendue, la vitesse de carbonylation. Exprimé différemment, le procédé selon l'invention permet d'atteindre une vitesse de carbonylation, exprimée en moles, au moins égale à celle obtenue, dans les mêmes conditions, avec un système catalytique ne mettant en jeu qu'un seul métal, alors que le nombre de moles total en métaux utilisés dans le procédé de l'invention, est inférieur.

Outre l'augmentation de la productivité, le fait d'obtenir des vitesses comparables en mettant en jeu un nombre de moles en catalyseur inférieur, constitue un avantage supplémentaire, d'ordre économique : celui de diminuer les coûts en catalyseur.

Par ailleurs, le procédé selon l'invention permet de réaliser efficacement la réaction de carbonylation, avec des teneurs en eau dans le milieu, inférieures à celles mises en oeuvre dans les procédés classiques, sans qu'il soit nécessaire d'ajouter audit milieu les quantités considérables, requises dans l'art antérieur, de stabilisant du catalyseur. Ainsi que cela a été indiqué plus haut, le procédé selon l'invention peut être mis en oeuvre en l'absence même de tels composés.

Mais d'autres avantages apparaîtront plus clairement à la lecture de la description qui va suivre.

La réaction selon l'invention est réalisée en phase liquide, par conséquent le système catalytique mis en oeuvre se présente sous forme soluble dans le milieu réactionnel.

Le système catalytique convenable à la réalisation de l'invention est à base d'au moins un composé du rhodium, d'au moins un composé de l'iridium et d'au moins un promoteur halogéné.

Les composés à base de rhodium et d'iridium utilisés habituellement dans les réactions de carbonylation, peuvent être mis en oeuvre dans le procédé selon l'invention.

Généralement, les composés à base de rhodium et d'iridium utilisés sont choisis parmi les complexes de coordination de ces métaux, solubles dans le milieu, dans les conditions de réaction. Plus particulièrement on utilise des complexes de coordination dont les ligands sont d'une part le monoxyde de carbone et d'autre part un halogène, comme le chlore, le brome ou plus particulièrement l'iode. Bien entendu, mettre en oeuvre des complexes solubles comprenant d'autres ligands que ceux mentionnés, comme notamment des ligands organo-phosphorés ou azotés, ne sortirait pas du cadre de la présente invention. Cependant, d'une façon avantageuse, la présente invention ne nécessite pas la mise en oeuvre de complexes de rhodium et d'iridium de ce type.

Ainsi, à titre d'exemples de complexes de coordination utilisés plus particulièrement dans la présente invention, on peut mentionner notamment les complexes du type Ir₄(CO)₁₂, Ir(CO)₂I₂⁻Q⁺, Ir(CO)₂Br₂⁻Q⁺, Rh₄(CO)₁₂, Rh(CO)₂I₂⁻Q⁺, Rh(CO)₂Br₂⁻Q⁺, ou encore des complexes à base des deux métaux comme Rh₃Ir(CO)₁₂, Rh₂Ir₂(CO)₁₂ ; formules dans lesquelles Q peut représenter notamment l'hydrogène, le groupe NR₄, PR₄, avec R choisi parmi l'hydrogène, un radical hydrocarboné.

Peuvent de même être mis en oeuvre dans le procédé selon l'invention, les composés choisis parmi les sels simples de ces éléments comme notamment IrI₃, IrBr₃, IrCl₃, IrI₃.4H₂O, IrBr₃.4H₂O, RhI₃, RhBr₃, RhCl₃, RhI_{3,} 4H₂O, RhBr_{3,} 4H₂O, ou encore le rhodium et l'iridium à l'état métallique.

Il est à noter que cette liste des composés à base de rhodium et d'iridium mentionnée ci-dessus, ne peut être considérée comme exhaustive, et qu'à titre d'exemples supplémentaires de composés des deux métaux précités, on pourra se référer aux brevets US 3 769 329 et US 3 772 380, dont l'enseignement est inclus ici.

La proportion d'un métal vis-à-vis de l'autre peut varier dans de larges limites. Ainsi, le rapport atomique rhodium/iridium dans le système catalytique selon l'invention est compris entre 0,01 et 99.

Généralement, la concentration totale en métal dans le milieu réactionnel est comprise entre 0,1 et 100 mmol/l de préférence entre 1 et 10 mmol/l.

Outre les composés à base de rhodium et d'iridium mentionnés ci-dessus, le système catalytique selon l'invention comprend un promoteur halogéné. Celui-ci peut se présenter sous la forme d'un halogène seul, ou en combinaison avec d'autres éléments tels que, par exemple, l'hydrogène, un radical alkyle en C₁-C₁₀, un radical acyle en C₁-C₁₀, ou encore un radical aryle en C₆-C₁₀.

L'halogène est en général choisi parmi le chlore, le brome ou l'iode l'iode étant préféré.

Selon un mode particulier de réalisation de l'invention, le promoteur mis en oeuvre comprend l'hydrogène ou un radical alkyle en C₁-C₁₀. Plus particulièrement, le promoteur utilisé dans l'invention comprend l'halogène et un radical alkyle en C₁-C₁₀.

De préférence, le radical précité du promoteur halogéné correspond au radical hydrocarboné du réactif carbonylé lors de la réaction selon l'invention.

A titre de composés halogénés susceptibles d'être utilisés en tant que promoteurs, on peut citer l'iode, l'acide iodhydrique, l'acide bromhydrique, l'iodure de méthyle, le bromure de méthyle, l'iodure d'éthyle, le diiodure 1,1-d'éthyle, le bromure de benzyle, l'iodure acétyle.

Selon une variante de l'invention, le promoteur halogéné est introduit dans le mélange réactionnel, partiellement ou totalement, sous la forme d'un précurseur. Dans un tel cas, ledit précurseur se présente généralement sous la forme d'un composé susceptible de libérer dans le milieu réactionnel, le radical hydrocarboné du promoteur halogéné précité, sous l'action d'un halogène ou de l'acide halogénhydrique notamment, ces derniers composés étant présents dans le milieu ou bien introduits dans ce but.

A titre d'exemples non limitatifs de précurseurs convenables, on peut citer les composés choisis parmi les alcools de formule (1) ROH; les éthers de formule (2) ROR' ou encore des esters de formules (3) R'COOR, utilisés seuls ou en mélange. Dans les formules précitées, les radicaux R et R', identiques ou différents, représentent chacun un radical hydrocarboné saturé en C₁-C₁₀ ou aromatique en C₆-C₁₀ ; le radical R correspondant au radical du promoteur halogéné.

Ainsi, le méthanol, l'éthanol, le propanol, le butanol, le diméthyl éther, l'acétate de méthyle, sont des précurseurs convenables dudit promoteur halogéné.

La quantité de promoteur halogéné présent dans le mélange réactionnel est comprise entre 0 exclu et 20 %, rapporté au poids total dudit mélange. De préférence, la teneur en promoteur halogéné est comprise entre 0 exclu et 15%.

Il est à noter que si le promoteur précité est introduit partiellement ou totalement, sous la forme d'un précurseur, la quantité en précurseur ou en mélange promoteur/précurseur est telle qu'elle permette d'obtenir une quantité équivalente à celle mentionnée ci-dessus.

La préparation d'acides carboxyliques, ou des esters correspondants, selon l'invention est effectuée à partir d'au moins un réactif présentant un atome de carbone en moins par rapport à l'acide ou à l'ester final produit. Ce réactif est choisi parmi les composés suivants, seuls ou en mélange :

R(OH)ₘ ; (1)

RX ; (2)

ROR' (3)

ROCOR' ; (4)

Formules dans lesquelles R et R', identiques ou différents, représentent chacun un radical hydrocarboné en C₁-C₁₀, R présentant n atomes de carbone ; X représente le chlore, le brome ou l'iode et m vaut 1 ou 2.

Ainsi la réaction de carbonylation selon l'invention peut être mise en oeuvre notamment en présence de méthanol, d'éthanol, de propanol, d'éthylène glycol, de 1,4- butanediol , d'iodure de méthyle, de bromure de méthyle, d'iodure d'éthyle, de diiodure 1,1-d'éthyle, d'oxyde d'éthylène, d'acétate de méthyle, d'acétate d'éthyle, d'iodure d'acétyle.

Selon un mode de réalisation préféré de l'invention, le réactif mis en jeu dans le procédé est choisi parmi les alcools monohydroxylés et les halogénures d'alkyle.

La teneur dans le mélange réactionnel en réactif est comprise entre 0 exclu et 40 % en poids, rapporté au poids total du milieu. De préférence, ladite teneur en réactif est comprise entre 0 exclu et 30 %.

L'autre réactif nécessaire à l'obtention d'un acide carboxylique est le monoxyde de carbone. Celui-ci peut être utilisé sous forme pure ou diluée dans des gaz tels que l'hydrogène, le méthane, le dioxyde de carbone ou tout autre type de gaz comme par exemple l'azote.

Selon un mode particulier de réalisation de l'invention, on utilise un monoxyde de carbone pur à au moins 99%.

La pression partielle en monoxyde de carbone varie habituellement entre 10 et 50 bar de préférence entre 10 et 20 bar.

La réaction de carbonylation selon l'invention est effectuée en outre, en présence d'eau. Ainsi que cela a été indiqué auparavant, le procédé selon l'invention permet d'obtenir une bonne productivité à de faibles teneurs en eau, sans perte de métal catalytique par précipitation.

Ainsi, le procédé, objet de l'invention, peut être réalisé dans une large gamme de concentration en eau dans le milieu réactionnel, soit comprise entre 0 exclu et 14 %, rapporté au poids total dudit milieu. Plus particulièrement la teneur en eau dans le milieu réactionnel est comprise entre 0 exclu et 10 %.

L'un des avantages remarquables de la présente invention, comme indiqué auparavant, réside dans le fait qu'il est possible d'effectuer la réaction de carbonylation sinon en l'absence de sels solubles d'iodures, au moins en ne les mettant en oeuvre que dans de faibles quantités.

Ainsi, la teneur en sels solubles d'iodures présents dans le milieu réactionnel est comprise entre 0 et 5 % en poids, rapporté au poids total dudit mélange. Il est rappelé ici que les sels solubles sont choisis parmi les composés classiques organiques ou minéraux et plus particulièrement parmi les iodures de métaux alcalins ou alcalino-terreux. De préférence, cette teneur est comprise entre 0 et 2 %.

Outre les composés et réactifs précédemment mentionnés, la réaction de carbonylation selon l'invention est réalisée en présence d'esters.

De préférence, l'ester utilisé correspond à la réaction de l'acide formé avec l'alcool présent dans le milieu, en tant que tel s'il est utilisé comme réactif, ou sous une forme masquée, si le réactif mis en oeuvre est un halogénure, un éther ou un ester.

Selon un mode de réalisation de l'invention, la teneur en ester est comprise entre 0 exclu et 40 % en poids, rapporté au poids du mélange réactionnel. Plus particulièrement cette teneur varie entre 0 exclu et 30 %.

Enfin la réaction de carbonylation est effectuée en présence d'un solvant. Avantageusement, le solvant mis en oeuvre dans le procédé selon l'invention est l'acide carboxylique ou l'ester formé. Bien entendu d'autres solvants peuvent être utilisés, comme notamment les composés inertes vis-à-vis du mélange réactionnel et présentant un point d'ébullition supérieur à celui de l'acide formé, tels que le benzène par exemple.

Habituellement la réaction de carbonylation est réalisée à une température comprise entre 150 et 250 °C.

La pression totale est généralement comprise entre 5 et 200 bar et entre 5 et 100 bar, de préférence.

Bien évidemment, le procédé selon l'invention peut être mis en oeuvre en discontinu comme en continu.

Dans le cas où celui-ci est mis en oeuvre en continu, les teneurs des différents éléments du mélange réactionnel, correspondant aux conditions de marche stable du procédé, figurent dans les gammes de concentrations données plus haut et sont maintenues telles, pendant le cours de la réaction.

Lors du démarrage de la réaction, les divers composants sont introduits dans un réacteur approprié, muni de moyens d'agitation suffisants pour assurer le transfert gaz-liquide. Il est à noter que si le réacteur comprend de préférence des moyens d'agitation mécanique du mélange réactionnel, il n'est pas exclus d'opérer sans de tels moyens, l'homogénéisation du mélange étant réalisée par l'introduction du monoxyde de carbone dans le réacteur.

Les composants du milieu réactionnel sont introduits sans ordre préférentiel, sous leur forme propre et/ou sous la forme d'un ou plusieurs précurseurs.

Il est à noter que ce procédé selon l'invention peut être mis en oeuvre industriellement dans les installations existantes, exploitant un catalyseur à base de rhodium.

Le procédé selon l'invention convient à la fabrication de tout type d'acides carboxyliques ou des esters correspondants, comprenant au minimum deux atomes de carbone. Ainsi, ledit procédé peut être mis en oeuvre pour préparer l'acide propionique à partir de l'éthanol, l'acide succinique à partir de l'oxyde d'éthylène, l'acide adipique à partir du 1,4-butanediol.

Toutefois, ce procédé convient tout particulièrement à l'obtention d'acide acétique ou d'acétate de méthyle, à partir notamment de méthanol, en présence d'acétate de méthyle, d'eau et d'iodure de méthyle comme promoteur halogéné.

Des exemples concrets mais non limitatifs de l'invention vont maintenant être présentés.

### EXEMPLE 1

Cet exemple a pour but d'illustrer la synergie créée lors de l'utilisation de rhodium et d'iridium par rapport à la mise en oeuvre de catalyseurs à base de l'un ou l'autre des métaux.

Le mode opératoire est le suivant :
Dans une ampoule en verre, on introduit successivement :
- 16,5 g d'acide acétique ;
- 2,3 g d'iodure de méthyle ;
- 0,5 g d'acétate de méthyle ;
- 1,3 g de méthanol ;
- 1,9 g d'eau.

Le catalyseur est introduit avant les composants mentionnés ci-dessus, sous la forme d'une solution de iodure de rhodium et/ou de chlorure d'iridium dans l'acide acétique. Ainsi, les solutions de rhodium et d'iridium mises en oeuvre comprennent respectivement 1,5 % et 1,75 % de métal dans l'acide acétique.

La quantité de rhodium et/ou d'iridium est par ailleurs ajustée de telle façon que la concentration totale en métal soit de 4 mmol/l.

Une fois les constituants chargés, l'ampoule est placée dans un autoclave, placé lui-même dans un four agité et raccordé à l'alimentation de CO sous pression.

L'autoclave, sous une pression initiale de 5 bar est porté à une température de 185°C et la pression totale est régulée à 30 bar.
L'autoclave est refroidi lorsqu'il reste environ 2 % d'acétate de méthyle dans le milieu réactionnel.

Le tableau ci-dessous rassemble les résultats obtenus :

**tableau 1**

| **ESSAIS** | **PROPORTION Rh/Ir (%/%)** | **V**_{**CARB**} **(mol/h.l)** |
|---|---|---|
| **A** | 100/0 | 6 |
| **B** | 75/25 | 7,5 |
| **C** | 50/50 | 6 |
| **D** | 25/75 | 6 |
| **E** | 0/100 | 4 |
| V_{CARB} représente la vitesse de carbonylation Les pourcentages indiqués sont exprimés en moles. | | |

Ce tableau montre bien que la vitesse de carbonylation du méthanol en présence des deux métaux est toujours supérieure à celle obtenue en ne mettant en oeuvre qu'un seul métal à la fois, dans des conditions où la teneur en eau est d'environ 10 % en poids du mélange réactionnel total et la teneur en iodure de méthyle d'environ 9% en poids.

### EXEMPLE 2

Cet exemple a pour but d'illustrer la synergie créée lors de l'utilisation de rhodium et d'iridium par rapport à la mise en oeuvre de catalyseurs à base de l'un ou l'autre des métaux dans des conditions différentes de celles de l'exemple 1.
Dans une ampoule en verre, on introduit donc successivement :
- 19,6 g d'acide acétique ;
- 1,15 g d'iodure de méthyle ;
- 0,5 g d'acétate de méthyle ;
- 1,3 g de méthanol ;
- 0,5 g d'eau.

Le catalyseur est introduit avant les composants mentionnés, sous la forme d'une solution de métal dans l'acide acétique pour chacun desdits métaux.

Les solutions de rhodium et d'iridium mises en oeuvre sont les mêmes que celles de l'exemple 1.

La quantité de rhodium et/ou d'iridium est en outre ajustée de telle façon que la concentration totale en métal soit de 4 mmol/l.

On opère selon la méthode décrite dans l'exemple 1. Le tableau ci-dessous rassemble les résultats obtenus :

**tableau 2**

| **ESSAIS** | **PROPORTION Rh/Ir (%/%)** | **V**_{**CARB**} **(mol/h.l)** |
|---|---|---|
| **A** | 100/0 | 2,2 |
| **B** | 75/25 | 3,5 |
| **C** | 50/50 | 3,5 |
| **D** | 25/75 | 3,6 |
| **E** | 0/100 | 3 |

Les vitesses de carbonylation obtenues montrent toujours l'effet de synergie des deux métaux mis en oeuvre simultanément pendant la réaction, dans des conditions où la teneur en eau est d'environ 5 % en poids (rapporté au poids total du mélange) et la teneur en iodure de méthyle d'environ 5 % en poids.

### EXEMPLE 3

Les essais suivants ont été réalisés en continu dans un autoclave munis de moyens d'introduction des réactifs nécessaires à la réaction. La solution réactionnelle comprend en moyenne 5,5 mmol/l de rhodium et d'iridium.

Le temps de séjour dans le réacteur est d'environ 10 minutes.

A la sortie de l'autoclave, le mélange est dégazé et refroidi.

La composition du mélange réactionnel est analysé par chromatographie en phase gazeuse.

La pression totale dans l'autoclave est de 30 bar et la température est maintenue à 190°C.

On opère de telle sorte que la composition du mélange réactionnel soit maintenue telle qu'indiquée dans le tableau 3.

La vitesse de carbonylation est obtenue par mesure du débit de consommation du CO en tenant compte en outre, de la quantité de CO engagée dans la formation de dioxyde de carbone.

**tableau 3**

| **ESSAIS** | **H**_{**2**}**O** | **CH**_{**3**}**OH** | **CH**_{**3**}**CO**_{**2**}**CH**_{**3**} | **CH**_{**3**}**I** | **CH**_{**3**}**CO**_{**2**}**H** | **Rh/Ir** | **V**_{**carb**} |
|---|---|---|---|---|---|---|---|
| **A** | 4,6 | 0,12 | 8,7 | 15 | 70 | 44/55 | 11,1 |
| **B** | 5,6 | 0,3 | 13 | 14,6 | 65 | 44/55 | 12,8 |
| **C** | 6,6 | 0,5 | 16,3 | 12,4 | 63 | 44/55 | 13,7 |

Dans ce tableau, les teneurs indiquées pour les divers constituants du mélange sont exprimées en poids, rapportées au poids total du mélange et la vitesse de carbonylation est exprimée en mol/h.l d'acide acétique formé.

## Revendications

1. Procédé de préparation d'acides carboxyliques comprenant (n+1) atomes de carbone, ou des esters correspondants, par mise en contact, dans le mélange réactionnel de monoxyde de carbone avec au moins un réactif choisi parmi les composés de formules :
R(OH)ₘ ; (1)
RX ; (2)
ROR' (3)
ROCOR' ; (4)
Formules dans lesquelles R et R', identiques ou différents, représentent chacun un radical hydrocarboné en C₁-C₁₀, R présentant n atomes de carbone : X représente le chlore, le brome ou l'iode et m vaut 1 ou 2, caractérisé en ce que l'on effectue la réaction en présence d'un système catalytique comprenant au moins un composé du rhodium, au moins un composé de l'iridium ou au moins un composé renfermant ces deux métaux, et au moins un promoteur halogéné.

2. Procédé selon la revendication précédente, caractérisé en ce que l'on met en oeuvre un système catalytique dans lequel le rapport atomique du rhodium par rapport à l'iridium, est compris entre 0,01 et 99.

3. Procédé selon l'une des revendications précédentes, caractérisé en ce que l'on met en oeuvre une concentration totale en rhodium et en iridium dans le milieu réactionnel comprise entre 0,1 et 100 mmol/l , et de préférence entre 1 et 10 mmol/l.

4. Procédé selon l'une des revendications précédentes, caractérisé en ce que l'on effectue la réaction en présence d'eau.

5. Procédé selon l'une des revendications précédentes, caractérisé en ce que l'on effectue la réaction en présence d'un teneur en eau comprise entre 0 exclu et 14 % en poids, rapporté au poids total du milieu réactionnel et de préférence comprise entre 0 exclu et 10 %.

6. Procédé selon l'une des revendications précédentes, caractérisé en ce que l'on effectue la réaction en présence d'un promoteur halogéné comprenant de l'hydrogène ou un radical alkyle en C₁-C₁₀, acyle en C₁-C₁₀ ou aryle en C₆-C₁₀ et de préférence un radical correspondant à celui du réactif précité.

7. Procédé selon l'une des revendications précédentes, caractérisé en ce que l'on effectue la réaction en présence d'une teneur en promoteur halogéné comprise entre 0 exclu et 20 % en poids, rapporté au poids total du mélange réactionnel et, de préférence comprise entre 0 exclu et 15 %.

8. Procédé selon l'une des revendications précédentes, caractérisé en ce que la réaction est effectuée en présence d'une teneur en réactif précité comprise entre 0 exclu et 40 % en poids, rapporté au poids total du mélange réactionnel et, de préférence comprise entre 0 exclu et 30 %.

9. Procédé selon l'une des revendications précédentes, caractérisé en ce que l'on effectue la réaction en présence d'un ester correspondant à la réaction de l'acide carboxylique présent avec le réactif précité, sous forme d'alcool ou sous une forme masquée de l'alcool.

10. Procédé selon l'une des revendications précédentes, caractérisé en ce que l'on effectue la réaction en présence d'une teneur en ester comprise entre 0 exclu et 40 % en poids, rapporté au poids total du mélange réactionnel et, de préférence comprise entre 0 exclu et 30 %.

11. Procédé selon l'une des revendications précédentes, caractérisé en ce que l'on effectue la réaction en utilisant l'acide carboxylique formé ou l'ester correspondant comme solvant.

12. Procédé selon l'une des revendications précédentes, caractérisé en ce que l'on effectue la réaction avec le méthanol, en présence d'iodure de méthyle, d'acétate de méthyle et d'eau, l'acide acétique étant choisi comme solvant.

## Claims

1. Process for the preparation of carboxylic acids comprising (n+1) carbon atoms, or corresponding esters, by bringing carbon monoxide, in the reaction mixture, in contact with at least one reagent selected from the compounds of formulae:
R(OH)m; (1)
RX; (2)
ROR'; (3)
ROCOR'; (4)
in which formulae R and R', which may be identical or different, each represent a C₁-C₁₀ hydrocarbon radical, R having n carbon atoms; X represents chlorine, bromine or iodine and m is 1 or 2, characterised in that the reaction is carried out in the presence of a catalytic system comprising at least one rhodium compound, at least one iridium compound or at least one compound containing these two metals, and at least one halogenated promoter.

2. Process according to the preceding claim, characterised in that a catalytic system is used in which the atomic ratio of rhodium relative to iridium is between 0.01 and 99.

3. Process according to one of the preceding claims, characterised in that a total concentration of rhodium and of iridium in the reaction medium is between 0.1 and 100 mmol/l, and preferably between 1 and 10 mmol/l, is used.

4. Process according to one of the preceding claims, characterised in that the reaction is carried out in the presence of water.

5. Process according to one of the preceding claims, characterised in that the reaction is carried out in the presence of a water content between 0 (exclusive of) and 14% by weight, relative to the total weight of the reaction medium, and preferably between 0 (exclusive of) and 10%.

6. Process according to one of the preceding claims, characterised in that the reaction is carried out in the presence of a halogenated promoter containing hydrogen or a C₁-C₁₀ alkyl, a C₁-C₁₀ acyl, or a C₆-C₁₀ aryl radical, and preferably a radical corresponding to that of the above-mentioned reagent.

7. Process according to one of the preceding claims, characterised in that the reaction is carried out in the presence of a content of halogenated promoter between 0 (exclusive of) and 20% by weight relative to the total weight of the reaction mixture, and preferably between 0 (exclusive of) and 15%.

8. Process according to one of the preceding claims, characterised in that the reaction is carried out in the presence of a content of above-mentioned reagent between 0 (exclusive of) and 40% by weight relative to the total weight of the reaction mixture, and preferably between 0 (exclusive of) and 30%.

9. Process according to one of the preceding claims, characterised in that the reaction is carried out in the presence of an ester corresponding to the reaction of the carboxylic acid present with the above-mentioned reagent, in the form of an alcohol or in a masked form of the alcohol.

10. Process according to one of the preceding claims, characterised in that the reaction is carried out in the presence of an ester content between 0 (exclusive of) and 40% by weight relative to the total weight of the reaction mixture, and preferably between 0 (exclusive of) and 30%.

11. Process according to one of the preceding claims, characterised in that the reaction is carried out by using the carboxylic acid formed or the corresponding ester as solvent.

12. Process according to one of the preceding claims, characterised in that the reaction is carried out with methanol, in the presence of methyl iodide, methyl acetate and water, acetic acid being selected as solvent.

## Patentansprüche

1. Verfahren zur Herstellung von Carbonsäuren, die (n+1) Kohlenstoffatome aufweisen, oder der entsprechenden Ester, bei dem man in der Reaktionsmischung Kohlenmonoxid in Kontakt bringt mit mindestens einem Reagens, ausgewählt aus den Verbindungen der Formeln:
R(OH)ₘ (1)
RX (2)
ROR' (3)
ROCOR' (4)
worin bedeuten:
R und R', die gleich oder verschieden sein können, jeweils einen C₁-C₁₀-Kohlenwasserstoffrest, wobei R n Kohlenstoffatome aufweist;
X Chlor, Brom oder Jod und
m die Zahl 1 oder 2,
dadurch gekennzeichnet, daß man die Reaktion in Gegenwart eines katalytischen Systems durchführt, das mindestens eine Rhodiumverbindung, mindestens eine Iridiumverbindung oder mindestens eine diese beiden Metalle enthaltende Verbindung und mindestens einen halogenierten Promotor umfaßt.

2. Verfahren nach dem vorhergehenden Anspruch, dadurch gekennzeichnet, daß man ein katalytisches System einsetzt, in dem das Atomverhältnis von Rhodium zu Iridium zwischen 0,01 und 99 liegt.

3. Verfahren nach einem der vorhergehenden Ansprüche, dadurch gekennzeichnet, daß man Rhodium und Iridium in dem Reaktionsmedium in einer Gesamtkonzentration einsetzt, die zwischen 0,1 und 100 mmol/l, vorzugsweise zwischen 1 und 10 mmol/l, liegt.

4. Verfahren nach einem der vorhergehenden Ansprüche, dadurch gekennzeichnet, daß man die Reaktion in Gegenwart von Wasser durchführt.

5. Verfahren nach einem der vorhergehenden Ansprüche, dadurch gekennzeichnet, daß man die Reaktion in Gegenwart eines Wassergehaltes durchführt, der zwischen 0 (wobei der Wert 0 ausgeschlossen ist) und 14 Gew.-%, vorzugsweise zwischen 0 (wobei der Wert 0 ausgeschlossen ist) und 10 Gew.-%, bezogen auf das Gesamtgewicht des Reaktionsmediums, liegt.

6. Verfahren nach einem der vorhergehenden Ansprüche, dadurch gekennzeichnet, daß man die Reaktion in Gegenwart eines halogenierten Promotors durchführt, der Wasserstoff oder einen C₁-C₁₀-Alkyl-, C₁-C₁₀-Acyl- oder C₆-C₁₀-Arylrest und vorzugsweise einen Rest aufweist, der demjenigen des vorgenannten Reagens entspricht.

7. Verfahren nach einem der vorhergehenden Ansprüche, dadurch gekennzeichnet, daß man die Reaktion in Gegenwart eines Gehaltes an halogeniertem Promotor durchführt, der zwischen 0 (wobei der Wert 0 ausgeschlossen ist) und 20 Gew.-%, vorzugsweise zwischen 0 (wobei der Wert 0 ausgeschlossen ist) und 15 Gew.-%, bezogen auf das Gesamtgewicht der Reaktionsmischung, liegt.

8. Verfahren nach einem der vorhergehenden Ansprüche, dadurch gekennzeichnet, daß die Reaktion in Gegenwart eines Gehaltes an dem obengenannten Reagens durchgeführt wird, der zwischen 0 (wobei der Wert 0 ausgeschlossen ist) und 40 Gew.-%, vorzugsweise zwischen 0 (wobei der Wert 0 ausgeschlossen ist) und 30 Gew.-%, bezogen auf das Gesamtgewicht der Reaktionsmischung, liegt.

9. Verfahren nach einem der vorhergehenden Ansprüche, dadurch gekennzeichnet, daß man die Reaktion in Gegenwart eines Esters durchführt, welcher der Reaktion der vorhandenen Carbonsäure mit dem obengenannten Reagens in Form eines Alkohols oder in einer mit dem Alkohol maskierten Form entspricht.

10. Verfahren nach einem der vorhergehenden Ansprüche, dadurch gekennzeichnet, daß man die Reaktion in Gegenwart eines Estergehaltes durchführt, der zwischen 0 (wobei der Wert 0 ausgeschlossen ist) und 40 Gew.-%, vorzugsweise zwischen 0 (wobei der Wert 0 ausgeschlossen ist) und 30 Gew.-%, bezogen auf das Gesamtgewicht der Reaktionsmischung, liegt.

11. Verfahren nach einem der vorhergehenden Ansprüche, dadurch gekennzeichnet, daß man die Reaktion durchführt unter Verwendung der gebildeten Carbonsäure oder des entsprechenden Esters als Lösungsmittel.

12. Verfahren nach einem der vorhergehenden Ansprüche, dadurch gekennzeichnet, daß man die Reaktion mit Methanol in Gegenwart von Methyljodid oder Methylacetat und Wasser durchführt, wobei die Essigsäure als Lösungsmittel gewählt wird.
